# EUROPÄISCHE PATENTSCHRIFT

(11) **EP 2 892 480 B1**
(45) Veröffentlichungstag und Bekanntmachung des Hinweises auf die Patenterteilung: **06.03.2019**
(21) Anmeldenummer: 13750694.5
(22) Anmeldetag: 16.08.2013
(51) Int. Cl.: A61F 13/00, A61F 13/08, D04B 21/18

(54) **WIRKWARE IN ORTHOPÄDISCHEN HILFSMITTELN**
WARP KNITTED FABRICS IN ORTHOPEDIC AIDS
STRUCTURE À MAILLES DANS DES ACCESSOIRES ORTHOPEDIQUES

(30) Priorität: 07.09.2012 DE 102012017722
(43) Veröffentlichungstag der Anmeldung: 15.07.2015
(73) Patentinhaber: Bauerfeind AG, 07937 Zeulenroda-Triebes (DE)
(72) Erfinder: STIER, Gerald, 07957 Langenwetzendorf (DE)
(74) Vertreter: Wohlfahrt, Jan Günther
(86) Internationale Anmeldenummer: PCT/EP2013/067154
(87) Internationale Veröffentlichungsnummer: WO 2014/037213

(56) Entgegenhaltungen:
- EP-A1- 0 071 818
- EP-A1- 1 895 036
- DE-A1- 3 519 677
- DE-C1- 10 101 530
- GB-A- 768 522
- US-A1- 2011 208 101
- US-A1- 2012 116 282

## Beschreibung

Die vorliegende Erfindung betrifft orthopädische Hilfsmittel, das heißt Bandagen und Orthesen, die mindestens eine Wirkware als Maschenware umfassen, wobei die Wirkware insbesondere eine Kettenwirkware ist, insbesondere eine polfädenfreie Kettenwirkware, mit einer offenen Struktur, die in Längs- und Querrichtung gleichzeitig dehnbar ist. Die vorliegende Erfindung betrifft auch die Verwendung solcher Wirkwaren, insbesondere Kettenwirkwaren in orthopädischen Hilfsmitteln, beispielsweise Bandagen und Orthesen, und Verfahren zur Herstellung dieser medizinischen Hilfsmittel.

Als Maschenwaren werden unter anderem Gewirke und Gestricke bezeichnet.

Es wird unter dem Ausdruck Gestrick, auch als Strickware bezeichnet, eine durch Stricken erzeugte Maschenware verstanden, also insbesondere kein Geflecht oder Gewebe. Ein Gestrick kann insbesondere ein Kompressionsgestrick sein. Strickwaren werden hergestellt, indem die Maschen mithilfe eines Fadens oder Garns nacheinander gebildet werden.

Gewirke, auch Gewirk oder Wirkwaren genannt, sind hingegen aus Fadensystemen durch Maschenbildung auf einer Wirkmaschine industriell hergestellte Stoffe. Sie gehören zu den Maschenwaren. Man unterscheidet zwischen Kulierwirkware und Kettenwirkware. Während beim Stricken oder Häkeln eine Masche neben der anderen hergestellt wird, der Faden also horizontal entlang einer Maschenreihe läuft, werden in einer Wirkware durch den Faden übereinander stehende Maschen gebildet und der Faden verläuft senkrecht und bildet mit dem benachbarten Faden ein Maschensteg. Eine Kettenwirkware wird mit vielen Fäden und mindestens ebenso vielen Nadeln hergestellt. Beim Kettenwirken laufen die Fäden vertikal und werden von den Nadeln ergriffen und durch die vorhergehende Maschenreihe gezogen.

Kettenwirkware wird beispielsweise in Form von Netzen eingesetzt, beispielsweise in Form von Gepäcknetzen in Automobilen. Die Netze können elastisch oder unelastisch ausgestaltet sein. Dabei spielt aber die Einhaltung genauer Parameter in Bezug auf die Elastizität, beispielsweise ein genau definiertes Kraft-Dehnungsverhalten der Netze, keine oder höchstens eine untergeordnete Rolle.

Die Erfindung hat sich die Aufgabe gestellt, orthopädische Hilfsmittel bereitzustellen, die neuartige und verbesserte Maschenwaren umfassen. Insbesondere soll die in den medizinischen, insbesondere orthopädischen Hilfsmitteln verwendete Maschenware die gewünschten Kraft-Dehnungsvorgaben aufweisen und dabei eine gute Atmungsaktivität haben. Die Maschenware soll auch so elastisch sein, dass eine gute Anpassung an die Körperform erreicht werden kann.

Die Erfindung hat sich ebenfalls die Aufgabe gestellt, neue Anwendungsbereiche für Wirkwaren, bevorzugt Kettenwirkwaren, insbesondere polfädenfreie Kettenwirkwaren in Netzform, bereitzustellen. Das der Erfindung zugrunde liegende technische Problem wird bevorzugt gelöst durch die Bereitstellung eines orthopädischen Hilfsmittels, Verwendungen und Verfahren gemäß des Hauptanspruchs und der Nebenansprüche und insbesondere durch ein medizinisches, erfindungsgemäß orthopädisches Hilfsmittel umfassend mindestens eine netzartige Wirkware nach Anspruch 1.Die mindestens eine Maschenware, erfindungsgemäß Wirkware, bevorzugt eine Kettenwirkware ist netzartig und bevorzugt als Netz ausgebildet. Die Maschenware, erfindungsgemäß die Wirkware, bevorzugt die Kettenwirkware ist also netzförmig, d.h. sie ist ein offenporiges Flächengebilde mit regelmäßigen Maschen, die als Öffnungen ausgebildet sind. Die Maschen beziehungsweise Öffnungen können beispielsweise rhombisch, quadratisch, sechseckig, rautenförmig oder wabenförmig ausgebildet sein. Eine Netzstruktur besteht bevorzugt aus einer Vielzahl von gleichen oder zumindest ähnlichen Teilstrukturen. Im Zusammenhang mit der vorliegenden Erfindung wird unter einer Teilstruktur eine Masche oder eine Öffnung und die die Masche oder Öffnung umgebenden und begrenzenden Fäden verstanden.

Im Zusammenhang mit der vorliegenden Erfindung wird unter einer offenen Struktur einer Maschenware, erfindungsgemäß Wirkware, bevorzugt Kettenwirkware insbesondere eine Netzstruktur verstanden, die eine Vielzahl an Löchern und Maschen aufweist, insbesondere an größeren Löcher, bevorzugt Löcher mit einer Breite und/oder Höhe von mindestens 2,0 mm, mehr bevorzugt mindestens 3,0 mm im gedehnten Zustand. Eine offene Struktur ist also insbesondere ein offenporiges Netz.

In dem vorliegenden Anmeldetext beziehen sich alle Angaben zur Höhe und/oder Breite der Löcher, Teilelemente und/oder Maschen auf die Höhe und/oder Breite der Löcher, Teilelemente und/oder Maschen im eingesetzten und verwendeten Zustand, also im gedehnten Zustand, der Wirkware im orthopädischen Hilfsmittel, sofern nichts anderes angegeben ist.

Im Zusammenhang mit der vorliegenden Erfindung wird unter dem Begriff "im gedehnten Zustand" derjenige Zustand der Wirkware verstanden, so wie die Wirkware in dem orthopädischen Hilfsmittel eingesetzt wird. Die Maschenware wird in dem orthopädischen Hilfsmittel erfindungsgemäß bevorzugt nicht im ungedehnten Zustand eingesetzt, sondern bevorzugt in einem vorgedehnten Zustand. Die Wirkware ist in dem orthopädischen Hilfsmittel bevorzugt nicht maximal gedehnt, sondern kann bevorzugt beim Anlegen des orthopädischen Hilfsmittels an den Körper eines Menschen oder Tieres weiter gedehnt werden.

In einer nicht-erfindungsgemäßen Ausführungsform der vorliegenden Erfindung ist die Maschenware eine Strickware, insbesondere ein Rund- oder Flachgestrick. In einer bevorzugten Ausführungsform ist die Maschenware ein Flachgestrick. In einer offenbarten Ausführungsform ist die Maschenware ein Rundgestrick. In einer offenbarten Ausführungsform ist die Maschenware dreidimensional. In einer offenbarten Ausführungsform ist die Maschenware ein dreidimensionales Flachgestrick, beispielsweise eine Bandage. In einer offenbarten Ausführungsform weist die Strickware Schussfäden, auch als Einleger und/oder Einlegefaden bezeichnet, auf.

Erfindungsgemäß ist die Maschenware eine Wirkware, insbesondere eine Kettenwirkware.

In einer bevorzugten Ausführungsform weist die Wirkware keine Polfäden auf.

In einer weiteren Ausführungsform der vorliegenden Erfindung wird ein medizinisches, erfindungsgemäß orthoädisches Hilfsmittel, umfassend eine, insbesondere netzartige, Maschenware, bereitgestellt, wobei die Maschenware eine Wirkware, insbesondere eine Kettenwirkware ist, die dadurch gekennzeichnet ist, dass die Wirkware keine Polfäden und eine offene Struktur aufweist, die gleichzeitig sowohl in Längsrichtung als auch in Querrichtung dehnbar ist.

In einer bevorzugten Ausführungsform weist die Wirkware einen Grundfaden und mindestens einen weiteren Faden, insbesondere Schussfaden, auf.

In einer bevorzugten Ausführungsform ist die Stärke des Grundfadens größer als die Stärke der mindestens einen weiteren Fadens, insbesondere des Schussfadens.

In einer anderen bevorzugten Ausführungsform ist die Stärke des Grundfadens geringer als die Stärke des mindestens einen weiteren Fadens, insbesondere des Schussfadens.

In einer bevorzugten Ausführungsform der Erfindung weist die Wirkware, bevorzugt die Kettenwirkware keine Polfäden auf, ist also polfädenfrei. Polfäden sind in Schlinge gelegte Fäden, die beispielsweise eine Dreidimensionalität erzeugen können.

Die Erfinder der vorliegenden Erfindung fanden, dass sich überraschenderweise auch eine Wirkware, insbesondere eine Kettenwirkware, insbesondere eine polfädenfreie Wirkware, insbesondere eine polfädenfreie Kettenwirkware, insbesondere wenn sie eine offene Struktur aufweist, als Maschenware in medizinischen Hilfsmitteln einsetzen lässt. Es zeigte sich überraschenderweise, dass die Wirkware, insbesondere die Kettenwirkware dabei besonders gut herkömmliche Maschenwaren ersetzen kann.

Die Erfinder der vorliegenden Erfindung fanden, dass die Maschenware, erfindungsgemäß Wirkware, bevorzugt die Kettenwirkware nicht nur so ausgestaltet werden kann, dass sie in Längs- und Querrichtung dehnbar, insbesondere gleichzeitig dehnbar ist, sondern auch, dass die Maschenware, erfindungsgemäß die Wirkware, bevorzugt die Kettenwirkware so ausgestaltet sein kann, dass sie benötigten Kraft-Dehnungsvorgaben in hoher Genauigkeit entspricht, dass sie also beim Anlegen einer spezifischen Kraft eine gewünschte und spezifische Dehnung aufweisen kann. Darüber hinaus ist die Maschenware, erfindungsgemäß die Wirkware, bevorzugt die Kettenwirkware durch die Maschen und Löcher, bevorzugt mit einer Breite und/oder Höhe von mindestens 2 mm und höchstens 10 mm im gedehnten Zustand, atmungsaktiv. Es zeigte sich, dass eine Maschenware, erfindungsgemäß Wirkware, bevorzugt eine Kettenwirkware eine deutlich bessere Atmungsaktivität aufweist als bisher im Stand der Technik verwendete Gestricke. Dadurch wird ein Wärmestau verhindert, so dass die Schweißbildung verringert wird. Die erfindungsgemäß verwendete Maschenware, erfindungsgemäß Wirkware, bevorzugt die Kettenwirkware weist eine Elastizität auf, die zu einer guten Anpassung an die Körperform führt, wobei sich die zweidimensionale Fläche dem dreidimensionalen Körper des Patienten optimal anpasst. Dies kann insbesondere durch die Ausgestaltung der Öffnungen als Rauten erreicht werden. Die Erfinder fanden auch, dass die Verwendung einer Maschenware, erfindungsgemäß Wirkware, bevorzugt einer Kettenwirkware zu einer verringerten Faltenbildung führen kann.

Die erfindungsgemäße Verwendung einer Wirkware, bevorzugt einer Kettenwirkware, insbesondere einer vorliegend definierten Kettenwirkware in einem medizinischen, erfindungsgemäß orthopädische Hilfsmittel, in Form einer Bandage oder einer Orthese führt also zu einer verbesserten Atmungsaktivität bei gleichzeitig genau vordefinierter Kompressionswirkung.

Durch den speziellen Aufbau der Wirkware, bevorzugt der Kettenwirkware können in vorteilhafter Weise neue Designeffekte, insbesondere Struktureffekte erzeugt werden, da Längsstreifen in unterschiedlichen Garnfarben möglich sind.

Eine solche Wirkware, bevorzugt eine Kettenwirkware ist in vorteilhafter Weise sowohl in Querrichtung als auch in Längsrichtung dehnbar.

In einer bevorzugten Ausführungsform ist die Wirkware, bevorzugt die Kettenwirkware im medizinischen, erfindungsgemäß orthopädischen Hilfsmittel in mindestens einer Richtung gedehnt. Die Wirkware, bevorzugt die Kettenwirkware ist also bevorzugt so in dem medizinischen, erfindungsgemäß orthopädischen Hilfsmittel eingesetzt, beispielsweise angeschweißt oder angeklebt, dass sie in der nicht verwendeten und ungedehnten Form des medizinischen, erfindungsgemäß orthopädischen Hilfsmittels in mindestens einer Richtung gedehnt, bevorzugt vorgedehnt ist.

Ein Fachmann könnte ohne weiteres die entsprechenden Parameter der Maschenware, erfindungsgemäß der Wirkware, bevorzugt der Kettenwirkware bestimmen und eine Maschenware, bevorzugt eine Strickware und/oder eine Wirkware, bevorzugt eine Kettenwirkware mit diesen Parametern herstellen.

Erfindungsgemäß ist das medizinische Hilfsmittel ein orthopädisches Hilfsmittel. Erfindungsgemäß ist das medizinische Hilfsmittel eine Bandage oder eine Orthese. In einer bevorzugten Ausführungsform ist das orthopädische Hilfsmittel eine Bandage. In einer alternativen Ausführungsform ist das orthopädische Hilfsmittel eine Orthese.

In einer bevorzugten Ausführungsform ist das orthopädische Hilfsmittel eine Rückenbandage. In einer alternativen Ausführungsform ist das orthopädische Hilfsmittel eine Armbandage oder Fußbandage.

In einer bevorzugten Ausführungsform ist die Wirkware, bevorzugt die Kettenwirkware als Bandagengrundträger ausgebildet.

In einer bevorzugten Ausführungsform ist die offene Struktur der Wirkware, bevorzugt der Kettenwirkware eine Netz-, Rauten- oder Wabenstruktur. In einer bevorzugten Ausführungsform ist die offene Struktur der Wirkware, bevorzugt der Kettenwirkware eine Rauten- oder Wabenstruktur. In einer bevorzugten Ausführungsform ist die offene Struktur der Wirkware, bevorzugt der Kettenwirkware eine Rautenstruktur.

In einer bevorzugten Ausführungsform haben die einzelnen Teilelemente der Struktur, insbesondere der Rautenstruktur jeweils eine Breite und/oder Höhe von mindestens 2 mm und höchstens 8 mm. In einer bevorzugten Ausführungsform haben die einzelnen Teilelemente der Struktur, insbesondere der Rautenstruktur jeweils eine Breite und/oder Höhe von mindestens 2 mm, insbesondere von mindestens 3 mm. In einer bevorzugten Ausführungsform haben die einzelnen Teilelemente der Struktur, insbesondere der Rautenstruktur jeweils eine Breite und/oder Höhe von höchstens 8 mm, insbesondere höchstens 6 mm. In einer bevorzugten Ausführungsform haben die einzelnen Teilelemente der Struktur, insbesondere der Rautenstruktur jeweils eine Breite und eine Höhe von mindestens 2 mm und höchstens 8 mm, insbesondere höchstens 6 mm. In einer bevorzugten Ausführungsform haben die einzelnen Teilelemente der Struktur, insbesondere der Rautenstruktur jeweils eine Breite von mindestens 2 mm und höchstens 6 mm. In einer bevorzugten Ausführungsform haben die einzelnen Teilelemente der Struktur, insbesondere der Rautenstruktur jeweils eine Höhe von mindestens 2 mm und höchstens 6 mm.

In einer bevorzugten Ausführungsform haben die einzelnen Teilelemente der Struktur, insbesondere der Rautenstruktur in der nicht verwendeten und ungedehnten Form des orthopädischen Hilfsmittels jeweils eine Breite und/oder Höhe von mindestens 0,5 mm und höchstens 5 mm, bevorzugt mindestens 0,7 mm und höchstens 4 mm, bevorzugt mindestens 0,9 mm und höchstens 3 mm, bevorzugt mindestens 1 mm und höchstens 3 mm im ungedehnten Zustand. In einer bevorzugten Ausführungsform haben die einzelnen Teilelemente der Struktur, insbesondere der Rautenstruktur in der verwendeten und gedehnten Form des orthopädischen Hilfsmittels jeweils eine Breite und/oder Höhe von mindestens 2 mm und höchstens 10 mm.

In einer bevorzugten Ausführungsform haben die Teilelemente der Struktur, insbesondere der Rautenstruktur oder Wabenstruktur in gedehntem Zustand, bevorzugt in maximal gedehntem Zustand jeweils eine Breite und/oder Höhe von mindestens 2 mm, bevorzugt mindestens 2 und höchstens 10 mm, bevorzugt mindestens 3 und höchstens 8 mm, bevorzugt mindestens 3 und höchstens 7 mm, bevorzugt mindestens 3 und höchstens 6 mm, bevorzugt mindestens 5 und höchstens 6 mm.

In einer bevorzugten Ausführungsform weisen die Teilelemente in Längs- und in Querrichtung die gleiche Ausdehnung auf. Bevorzugt sind im gedehnten Zustand die Höhe und die Breite der Teilelemente der in der Wirkware vorliegenden Struktur ungefähr gleich groß, bevorzugt gleich groß.

In einer bevorzugten Ausführungsform haben die einzelnen Teilelemente der Struktur, insbesondere der Rautenstruktur in der nicht verwendeten und ungedehnten Form jeweils eine Breite und/oder Höhe von mindestens 0,5 mm und in der verwendeten und gedehnten Form im medizinischen Hilfsmittel eine Breite und/oder Höhe von höchstens 8 mm.

Teilelemente der Struktur, insbesondere der Rautenstruktur mit den hier genannten Größen führen in vorteilhafter Weise zur Verminderung oder gar Verhinderung einer Ödembildung.

In einer bevorzugten Ausführungsform enthält die Wirkware, bevorzugt die Kettenwirkware mindestens einen gummielastischen Faden.

In einer bevorzugten Ausführungsform enthält die Wirkware, bevorzugt die Kettenwirkware mindestens einen gummielastischen Faden und mindestens ein Umwindegarn.

In einer bevorzugten Ausführungsform besteht die Wirkware, bevorzugt die Kettenwirkware aus mindestens einem gummielastischen Faden und mindestens einem Umwindegarn. In einer bevorzugten Ausführungsform besteht die Wirkware, bevorzugt die Kettenwirkware aus gummielastischem Faden und Umwindegarn.

In einer bevorzugten Ausführungsform besteht eine Komponente der Wirkware, bevorzugt der Kettenwirkware aus einem doppelt umwundenen gummielastischen Faden. In einer bevorzugten Ausführungsform besteht jeweils ein Teilelement der Wirkware, bevorzugt der Kettenwirkware aus einem doppelt umwundenen gummielastischen Faden. In einer bevorzugten Ausführungsform besteht die Wirkware, bevorzugt die Kettenwirkware aus einem doppelt umwundenen gummielastischen Faden.

Umwundene Gummifäden führen in vorteilhafter Weise zu einer guten Längselastizität der Wirkware, bevorzugt der Kettenwirkware.

In einer bevorzugten Ausführungsform besteht der gummielastische Faden aus einem thermoplastischen Fadenmaterial.

In einer bevorzugten Ausführungsform besteht die Wirkware, bevorzugt die Kettenwirkware aus einem Faden, bevorzugt einem Kettfaden mit einer Feinheit oder Stärke von mindestens 700 dtex. In einer bevorzugten Ausführungsform besteht die Wirkware, bevorzugt die Kettenwirkware aus einem Faden, bevorzugt einem Kettfaden mit einer Feinheit oder Stärke von höchstens 10,5 ktex. In einer bevorzugten Ausführungsform besteht die Wirkware, bevorzugt die Kettenwirkware aus einem Kettfaden mit einer Feinheit oder Stärke von mindestens 700 dtex bis höchstens 10,5 ktex. In einer bevorzugten Ausführungsform besteht die Wirkware, bevorzugt die Kettenwirkware aus einem Faden, bevorzugt einem Kettfaden mit einer Feinheit oder Stärke von mindestens 850 dtex bis höchstens 1050 dtex. In einer bevorzugten Ausführungsform besteht die Wirkware, bevorzugt die Kettenwirkware aus einem Faden, bevorzugt einem Kettfaden mit einer Feinheit oder Stärke von mindestens 900 dtex bis höchstens 999 dtex. In einer bevorzugten Ausführungsform besteht die Maschenware, bevorzugt die Strickware und/oder die Wirkware, bevorzugt die Kettenwirkware aus einem Faden, bevorzugt einem Kettfaden mit einer Feinheit oder Stärke von mindestens 850 dtex. In einer bevorzugten Ausführungsform besteht die Wirkware, bevorzugt die Kettenwirkware aus einem Faden, bevorzugt einem Kettfaden mit einer Feinheit oder Stärke von etwa 940 dtex.

Es zeigte sich, dass sich durch die Wahl des Materials und/oder der Teilelemente der Wirkware, bevorzugt der Kettenwirkware das Kraft-Dehnungsverhalten der Kettenwirkware genau definiert und eingestellt werden konnte.

In einer bevorzugten Ausführungsform hat die Wirkware, bevorzugt die Kettenwirkware etwa 30% Dehnung bei einer angelegten Kraft von 1,0 bis 5,0 N/cm, bevorzugt etwa 2,5 N/cm. In einer besonders bevorzugten Ausführungsform hat die Wirkware, bevorzugt die Kettenwirkware 30% Dehnung bei einer angelegten Kraft von 1,0 bis 5,0 N/cm, bevorzugt 2,5 N/cm.

In einer bevorzugten Ausführungsform ist die Wirkware, bevorzugt die Kettenwirkware in dem medizinischen Hilfsmittel in mindestens einer Richtung, bevorzugt in Längsrichtung und/oder Querrichtung, um mindestens 10% und höchstens 90%, bevorzugt mindestens 15% und höchstens 80%, bevorzugt mindestens 15% und höchstens 70%, bevorzugt mindestens 20% und höchstens 50%, bevorzugt mindestens 20% und höchstens 40%, im Vergleich zum ungedehnten Zustand der Wirkware gedehnt.

In einer bevorzugten Ausführungsform dient die Wirkware, bevorzugt die Kettenwirkware als Kompressionselement in dem medizinischen, erfindungsgemäß orthopädischen Hilfsmittel.

In einer bevorzugten Ausführungsform ist an der Wirkware, bevorzugt der Kettenwirkware mindestens ein Formelement, ein Stützelement und/oder eine Zone unelastischen Materials angebracht. In einer bevorzugten Ausführungsform ist an der Wirkware, bevorzugt an der Kettenwirkware mindestens ein Formelement, ein Stützelement oder eine Zone unelastischen Materials angebracht.

In einer bevorzugten Ausführungsform ist das mindestens eine Formelement, Stützelement oder mindestens ein unelastisches Element auf die Wirkware, bevorzugt die Kettenwirkware aufgeschweißt, aufgenäht, aufgeklettet, aufgenietet oder aufgeklebt. In einer bevorzugten Ausführungsform ist das mindestens eine Formelement, Stützelement oder mindestens ein unelastisches Element auf die Wirkware, bevorzugt die Kettenwirkware aufgeschweißt oder aufgeklebt. In einer bevorzugten Ausführungsform ist das mindestens eine Formelement, Stützelement oder mindestens ein unelastisches Element auf die Wirkware, bevorzugt die Kettenwirkware aufgeschweißt. Es zeigte sich, dass die hier offenbarte Maschenware, erfindungsgemäß die Wirkware, bevorzugt die Kettenwirkware bei Verwendung eines thermoplastischen Fadenmaterials gut thermisch verarbeitbar ist, beispielsweise mittels Ultraschallschneiden und/oder Heißschnitt und/oder mittels Ultraschallschweißen und/oder Hochfrequenz-Schweißen.

Die Erfindung löst das ihr zugrunde liegende technische Problem auch durch die Verwendung einer Maschenware, erfindungsgemäß einer erfindungsgemäßen Wirkware, bevorzugt Kettenwirkware in einem medizinischen, erfindungsgemäß orthopädischen Hilfsmittel.

In einer bevorzugten Ausführungsform wird die Wirkware, bevorzugt die Kettenwirkware in einer Bandage oder Orthese verwendet. In einer bevorzugten Ausführungsform wird die Wirkware, bevorzugt die Kettenwirkware in einer Bandage verwendet. In einer bevorzugten Ausführungsform wird die Wirkware, bevorzugt die Kettenwirkware in einer Bandage als Bandagengrundträger verwendet.

In einer bevorzugten Ausführungsform wird die die Wirkware, bevorzugt die Kettenwirkware als Kompressionselement in dem orthopädischen Hilfsmittel verwendet.

Die vorliegende Erfindung betrifft auch Verfahren zur Herstellung eines medizinischen, erfindungsgemäß orthopädischen Hilfsmittels, enthaltend die Schritte: a) Vorspannen einer Wirkware, bevorzugt einer Kettenwirkware; und b) Aufbringen des mindestens einen Formelements, Stützelements und/oder unelastisches Elements auf die vorgespannte Wirkware, bevorzugt Kettenwirkware.

Erfindungsgemäß handelt es sich bei dem medizinischen Hilfsmittel um ein orthopädisches Hilfsmittel. Erfindungsgemäß handelt es sich bei der Wirkware, bevorzugt Kettenwirkware um eine weiter oben beschriebene Wirkware, bevorzugt Kettenwirkware.

In einer bevorzugten Ausführungsform wird in Schritt b) das mindestens eine Formelement, Stützelement und/oder unelastisches Element auf die vorgespannte Wirkware, bevorzugt Kettenwirkware aufgeschweißt, aufgenäht, aufgeklettet, aufgenietet oder aufgeklebt. In einer bevorzugten Ausführungsform wird in Schritt b) das mindestens eine Formelement, Stützelement oder unelastisches Element auf die vorgespannte Wirkware, bevorzugt Kettenwirkware aufgeschweißt, aufgenäht, aufgeklettet, aufgenietet oder aufgeklebt. In einer bevorzugten Ausführungsform wird in Schritt b) das mindestens eine Formelement, Stützelement oder unelastisches Element auf die vorgespannte Wirkware, bevorzugt Kettenwirkware aufgeschweißt, oder aufgeklebt. In einer bevorzugten Ausführungsform wird in Schritt b) das mindestens eine Formelement, Stützelement oder unelastisches Element auf die vorgespannte Wirkware, bevorzugt Kettenwirkware aufgeschweißt, beispielsweise durch Ultraschallschweißverfahren aufgeschweißt.

Schritt a) können natürlich noch weitere Schritte vorgelagert sein, zum Beispiel maschinelles Herstellen der Wirkware, bevorzugt der Kettenwirkware und Zustanzen oder Zuschneiden der Wirkware, bevorzugt der Kettenwirkware, zum Beispiel durch Heißzuschnitt oder Laserzuschnitt.

Bevorzugte Ausführungsformen ergeben sich auch aus den Unteransprüchen.

Die Erfindung wird nachfolgend anhand der in den Zeichnungen dargestellten Ausführungsbeispiele näher erläutert, ohne dass eine Beschränkung der Erfindung erfolgt.

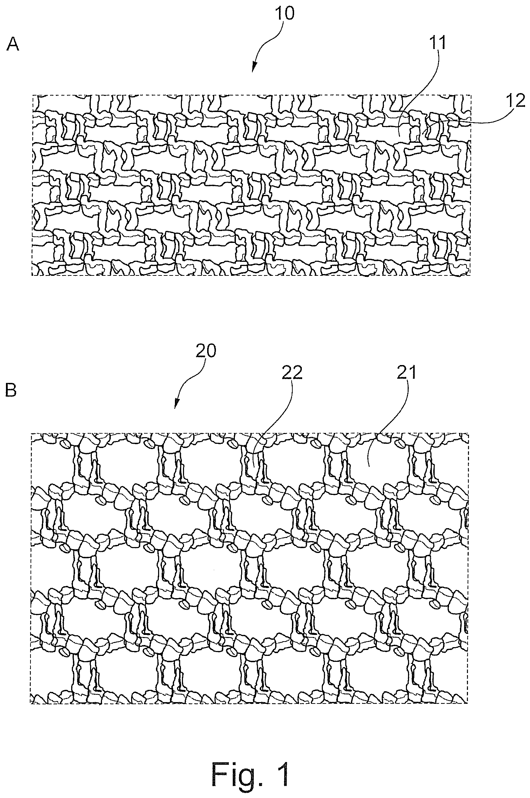

Dabei zeigt die Figur 1 einen Ausschnitt einer erfindungsgemäßen Kettenwirkware mit mehreren Teilelementen. In A ist die Kettenwirkware in einem ungedehnten Zustand gezeigt. In B ist die Kettenwirkware in einem gedehnten Zustand gezeigt.

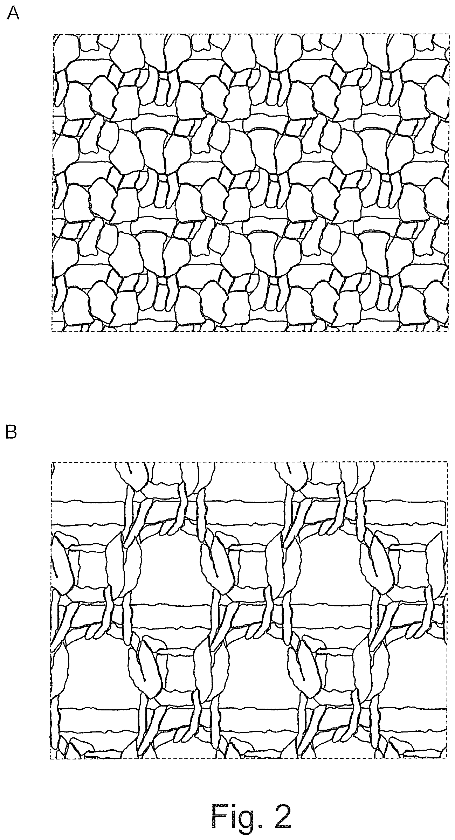

Figur 2 zeigt einen Ausschnitt einer nicht-erfindungsgemäßen Strickware aus mehreren Teilelementen. In A ist die Strickware in einem ungedehnten Zustand gezeigt. In B ist die Strickware in einem gedehnten Zustand gezeigt.

### Beispiel:

Es wurde eine Kettenwirkware aus einem doppelt umwundenen Rohgummifaden maschinell hergestellt. Der verwendete maschenbildende Faden hatte einen Titer von 940 dtex. Von dieser Kettenwirkware (10, 20) ist in der Figur ein Ausschnitt zu sehen. Die Kettenwirkware (10, 20) ist netzförmig. Die Kettenwirkware (10, 20) besteht aus einer Vielzahl von Teilelementen, wobei jedes Teilelement aus einer Öffnung (11, 21) und dem die Öffnung (11, 21) umgebenden Faden (12, 22) besteht, der die Öffnung (10, 20) auch gleichzeitig begrenzt und somit die Größe der Öffnung vorgibt. In A ist die Kettenwirkware (10) nicht gedehnt. In B ist die Kettenwirkware (20) mit einer bestimmten Kraft gedehnt. Durch die Dehnung der Kettenwirkware (20) öffnet sich die Struktur (22), so dass sich die Öffnungen (21) in Dehnungsrichtung vergrößern.

Die Kettenwirkware kann beispielsweise nun in die passende Größe zurechtgeschnitten werden und als Grundträger einer Rückenbandage fungieren. Dabei können weitere Elemente der Rückenbandage an die Kettenwirkware angebracht werden. Beispielsweise können Formelemente angeschweißt werden, so dass die Kettenwirkware in der ungedehnten Grundform der Rückenbandage in einer Richtung gedehnt ist.

## Patentansprüche

1. Orthopädisches Hilfsmittel umfassend mindestens eine netzartige Maschenware, **dadurch gekennzeichnet,**
**dass** die Maschenware eine offene Struktur aufweist, die gleichzeitig sowohl in Längsrichtung als auch in Querrichtung dehnbar ist, und wobei die Maschenware in einem gedehnten Zustand in dem orthopädischen Hilfsmittel eingesetzt wird und wobei die Teilelemente der Struktur jeweils eine Breite von mindestens 2,0 mm und höchstens 10,0 mm in dem gedehnten Zustand haben, wobei die Maschenware eine Wirkware ist und wobei das orthopädische Hilfsmittel eine Bandage oder eine Orthese ist.

2. Orthopädisches Hilfsmittel nach Anspruch 1, wobei die Wirkware eine Kettenwirkware ist.

3. Orthopädisches Hilfsmittel nach Anspruch 1, wobei die Wirkware keine Polfäden aufweist.

4. Orthopädisches Hilfsmittel nach einem der vorhergehenden Ansprüche, wobei die Maschenware im medizinischen Hilfsmittel in mindestens einer Richtung gedehnt ist.

5. Orthopädisches Hilfsmittel nach einem der vorhergehenden Ansprüche, wobei das orthopädische Hilfsmittel eine Rückenbandage ist.

6. Orthopädisches Hilfsmittel nach einem der vorhergehenden Ansprüche, wobei die offene Struktur eine Netz-, Rauten- oder Wabenstruktur ist.

7. Orthopädisches Hilfsmittel nach einem der Ansprüche 1 bis 6, wobei die Wirkware mindestens einen gummielastischen Faden und ein Umwindegarn enthält.

8. Orthopädisches Hilfsmittel nach einem der Ansprüche 1 bis 7, wobei die Kettenwirkware aus einem Kettfaden mit einer Feinheit oder Stärke von mindestens 700 dtex bis höchstens 10,5 ktex besteht.

9. Orthopädisches Hilfsmittel nach einem der vorhergehenden Ansprüche, wobei an der Maschenware mindestens ein Formelement, ein Stützelement und/oder eine Zone unelastischen Materials angebracht ist.

10. Verfahren zur Herstellung eines orthopädischen Hilfsmittels nach Anspruch 9, enthaltend die Schritte:
a. Vorspannen der Wirkware und
b. Aufbringen des mindestens einen Formelements, Stützelements und/oder unelastisches Elements auf die vorgespannte Wirkware.

11. Verfahren nach Anspruch 10, wobei in Schritt b) das mindestens eine Formelement, Stützelement und/oder unelastisches Element auf die vorgespannte Wirkware aufgeschweißt, aufgenäht, aufgeklettet, aufgenietet oder aufgeklebt wird.

12. Verwendung einer Wirkware aus einem der Ansprüche 1 bis 9 in einem orthopädischen Hilfsmittel, insbesondere in einer Bandage als Bandagengrundträger, wobei die Wirkware eine offene Struktur aufweist, die gleichzeitig sowohl in Längsrichtung als auch in Querrichtung dehnbar ist, und wobei die Wirkware in einem gedehnten Zustand in dem orthopädischen Hilfsmittel eingesetzt wird, und wobei die Teilelemente der Struktur jeweils eine Breite von mindestens 2,0 mm und höchstens 10,0 mm in gedehntem Zustand haben.

13. Verwendung nach Anspruch 12, wobei die Wirkware als Kompressionselement in dem othopädischen Hilfsmittel verwendet wird.

## Claims

1. An orthopedic aid comprising at least one mesh-like knit wear, **characterized in**
**that** the knit wear has an open structure that can be stretched simultaneously in both the longitudinal direction and the transverse direction, and wherein the knit wear is used in a stretched state in the orthopedic aid, and wherein each of the sub-elements of the structure have a width of at least 2.0 mm and at most 10.0 mm in the stretched state, wherein the knit wear is a knitted fabric and wherein the orthopedic aid is a bandage or an orthosis.

2. The orthopedic aid according to claim 1, wherein the knitted fabric is a warp-knitted fabric.

3. The orthopedic aid according to claim 1, wherein the knitted fabric has no pile threads.

4. The orthopedic aid according to any one of the preceding claims, wherein the knit wear is stretched in at least one direction in the medical aid.

5. The orthopedic aid according to any one of the preceding claims, wherein the orthopedic aid is a back bandage.

6. The orthopedic aid according to any one of the preceding claims, wherein the open structure is a mesh structure, rhombic structure or honeycomb structure.

7. The orthopedic aid according to any one of claims 1 to 6, wherein the knitted fabric contains at least one rubbery-elastic thread and one wrapped thread.

8. The orthopedic aid according to any one of claims 1 to 7, wherein the warp-knitted fabric consists of a warp thread having a gauge or thickness of at least 700 dtex to at most 10.5 ktex.

9. The orthopedic aid according to any one of the preceding claims, wherein at least one shaping element, one support element and/or one zone of inelastic material is attached to the knit wear.

10. Process for producing an orthopedic aid according to claim 9, comprising the following steps:
a. prestressing the knitted fabric; and
b. applying the at least one shaping element, support element and/or inelastic element to the prestressed knitted fabric.

11. The process according to claim 10, wherein in step b) the at least one shaping element, support element and/or inelastic element is welded, stitched, attached by hook-and-loop tape, riveted or glued to the prestressed knitted fabric.

12. The use of a knitted fabric of any one of claims 1 to 9 in an orthopedic aid, in particular in a bandage as a base support for a bandage, wherein the knitted fabric has an open structure stretchable simultaneously in both a longitudinal direction and a transverse direction, and wherein the knitted fabric is used in a stretched state in the orthopedic aid, and wherein the sub-elements of the structure each have a width of at least 2.0 mm and at most 10.0 mm in the stretched state.

13. The use according to claim 12, wherein the knitted fabric is used as a compression element in the orthopedic aid.

## Revendications

1. Aide orthopédique comprenant au moins un tissu maillé sous forme de filet, **caractérisée en ce**
**que** le tissu maillé présente une structure ouverte qui est expansible soit dans la direction longitudinale soit dans la direction transversale, et le tissu maillé étant utilisée dans l'aide orthopédique dans un état expandu et les éléments partiels de la structure chacun présentant une largeur d'au moins 2,0 mm et au plus 10,0 mm dans l'état expandu, le tissu maillé étant un tissu tricoté et l'aide orthopédique étant un bandage ou une orthèse.

2. Aide orthopédique selon la revendication 1, dans laquelle le tissu tricoté est un tissu tricoté à maille jeté.

3. Aide orthopédique selon la revendication 1, dans laquelle le tissu tricoté ne présente pas de fils de poil.

4. Aide orthopédique selon l'une quelconque des revendications précédentes, dans laquelle le tissu maillé dans l'aide médicale est expandu dans au moins une direction.

5. Aide orthopédique selon l'une quelconque des revendications précédentes, l'aide orthopédique étant un bandage dorsal.

6. Aide orthopédique selon l'une quelconque des revendications précédentes, dans laquelle la structure ouverte est une structure en filet, en losange ou en nid d'abeille.

7. Aide orthopédique selon l'une quelconque des revendications 1 à 6, dans laquelle le tissu tricoté contient au moins un fil élastique et un fil guipé.

8. Aide orthopédique selon l'une quelconque des revendications 1 à 7, dans laquelle le tissu tricoté à maille jeté consiste en un fil de chaîne avec une finesse ou épaisseur d'au moins 700 dtex et au plus 10,5 ktex.

9. Aide orthopédique selon l'une quelconque des revendications précédentes, dans laquelle au moins un élément moulé, un élément de support et/ou une zone en matériau inélastique sont fixés sur le tissu maillé.

10. Procédé pour la fabrication d'une aide orthopédique selon la revendication 9, comprenant les étapes consistant à :
a. précontraindre le tissu tricoté et
b. appliquer le au moins un élément moulé, élément de support et/ou élément inélastique sur le tissu tricoté précontraint.

11. Procédé selon la revendication 10, dans lequel, dans l'étape b), le au moins un élément moulé, élément de support et/ou élément inélastique est fixé sur le tissu tricoté précontraint par soudage, couture, par velcro, par rivetage ou par collage.

12. Utilisation d'un tissu tricoté selon l'une quelconque des revendications 1 à 9 dans une aide orthopédique, notamment dans un bandage en tant que support de base du bandage, le tissu tricoté ayant une structure ouverte qui est expansible soit dans la direction longitudinale soit dans la direction transversale, et le tissu tricoté étant utilisée dans l'aide orthopédique dans un état expandu, et les éléments partiels de la structure chacun présentant une largeur d'au moins 2,0 mm et au plus 10,0 mm dans l'état expandu.

13. Utilisation selon la revendication 12, dans laquelle le tissu tricoté est utilisé dans l'aide orthopédique en tant qu'élément de compression.
